(19) Europäisches Patentamt
European Patent Office
Office européen des brevets

(11) EP 2 455 116 B1

(12) EUROPEAN PATENT SPECIFICATION

(45) Date of publication and mention
of the grant of the patent:
**20.04.2016 Bulletin 2016/16**

(51) Int Cl.:
***A61M 1/36*** (2006.01)

(21) Application number: **11189430.9**

(22) Date of filing: **16.11.2011**

(54) **Device for the continuous monitoring of characteristic blood quantities in extracorporeal circulation circuit**

Vorrichtung zur kontinuierlichen Überwachung von charakteristischen Eigenschaften von Blut im extrakorporalen Kreislauf

Dispositif pour la surveillance continue de propriétés caractéristiques de sang dans un circuit de circulation extracorporel

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.11.2010 IT MI20102157**

(43) Date of publication of application:
**23.05.2012 Bulletin 2012/21**

(73) Proprietor: **Eurosets S.r.l.**
**41036 Medolla (MO) (IT)**

(72) Inventors:
• **Ghelli, Nicola**
**40018 S. Pietro in Casale (BO) (IT)**
• **Foschieri, Stefano**
**41036 Medolla (MO) (IT)**
• **Petralia, Antonio**
**48022 Lugo (RA) (IT)**

(74) Representative: **Zoli, Filippo**
**BRUNACCI & PARTNERS S.r.l.**
**Via Scaglia Est, 19-31**
**41126 Modena (IT)**

(56) References cited:
**US-A- 4 401 431**     **US-A1- 2002 085 952**
**US-A1- 2004 220 509**     **US-A1- 2009 099 498**
**US-B1- 6 228 033**

**Description**

The invention relates to a device for the continuous monitoring of characteristic blood quantities in extracorporeal circulation circuit.

It is known that during heart-surgery operations an extracorporeal blood circulation is established with the main object of irrorating the vital organs, i.e., their supply with oxygenated blood, to ensure they work correctly and, for the purpose, one of the appliances included in the extracorporeal circuit consists of an oxygenator device in which the blood coming from the venous line of withdrawal from the patient is enriched with oxygen before being introduced into the arterial return-to-the-patient line. To keep the operation of the assembly under control so as to avoid insufficient oxygen reaching the tissues, today frequent samples are taken of the arterial and venous blood and these are analysed thanks to the presence in the operating theatre of an appliance called blood gas analyser, but it has been demonstrated that this method is unable to provide any reliable data as regards the condition of irroration of the patient. Systems have been proposed for monitoring blood characteristics comprising a micro processor which receives and processes data measured by sensors and data entered by operators, but these systems have proven to be lacking. US 4,401,431, for example, discloses a monitoring system for extracorporal blood oxygenation. The aim of the present invention is then to make a device able to perform a continuous monitoring of a plurality of characteristic blood quantities, providing operators with information in two representation forms, numerical and graphical, able to fully guide them during operations to achieve successful results.

The proposed aim is achieved by a device for the continuous monitoring of characteristic blood quantities in extracorporeal circulation circuit, according to the invention, characterized by the fact that it comprises the characteristics indicated in the following claims.

Further characteristics and advantages will become clearer from the description of two preferred but not exclusive embodiments of the invention, including but not limited to those shown in the attached drawings wherein:

- The figures from 1 to 20 relate to a first embodiment of the invention, as will be specified in detail during the course of the description;

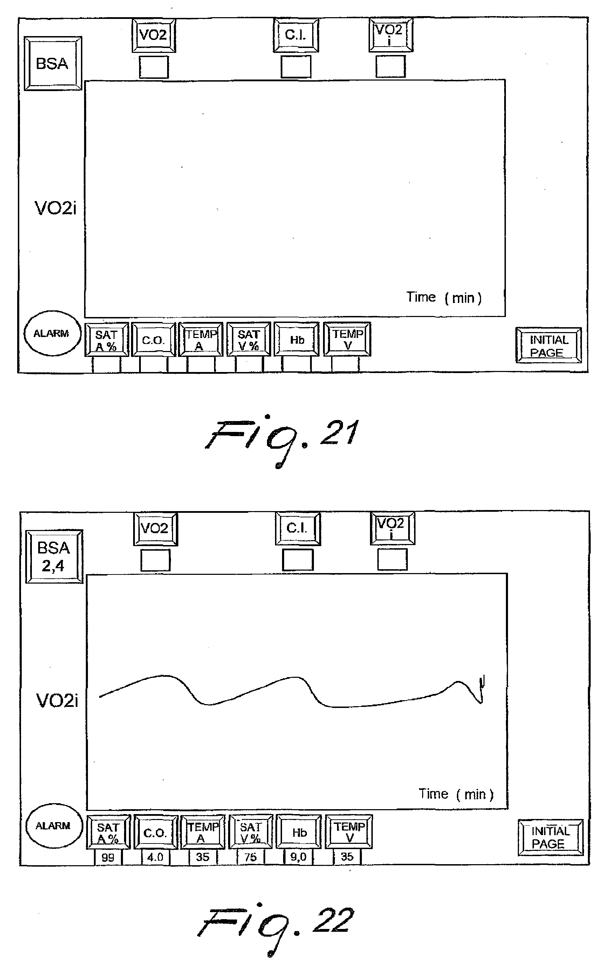

- The figures 21 and 22 relate to a variation.

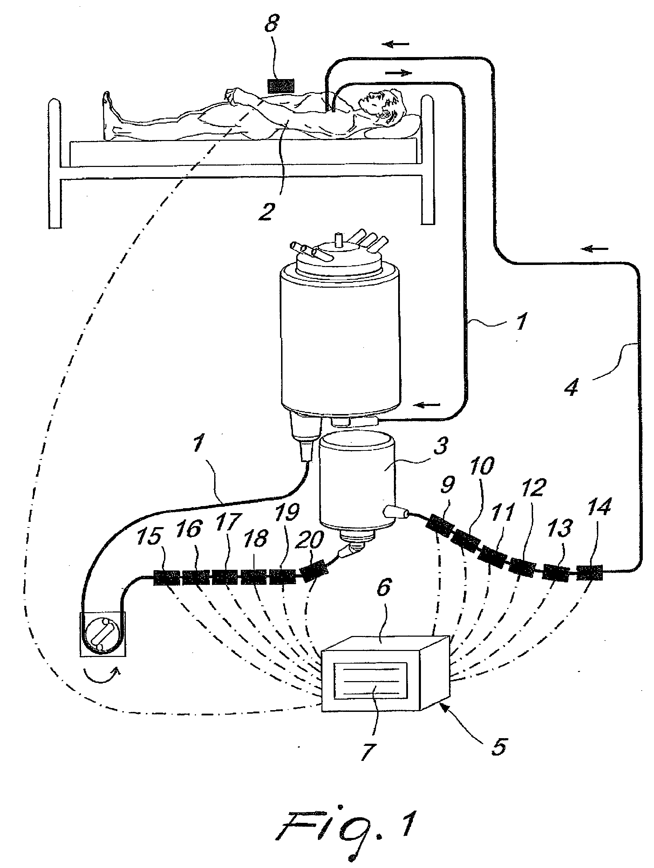

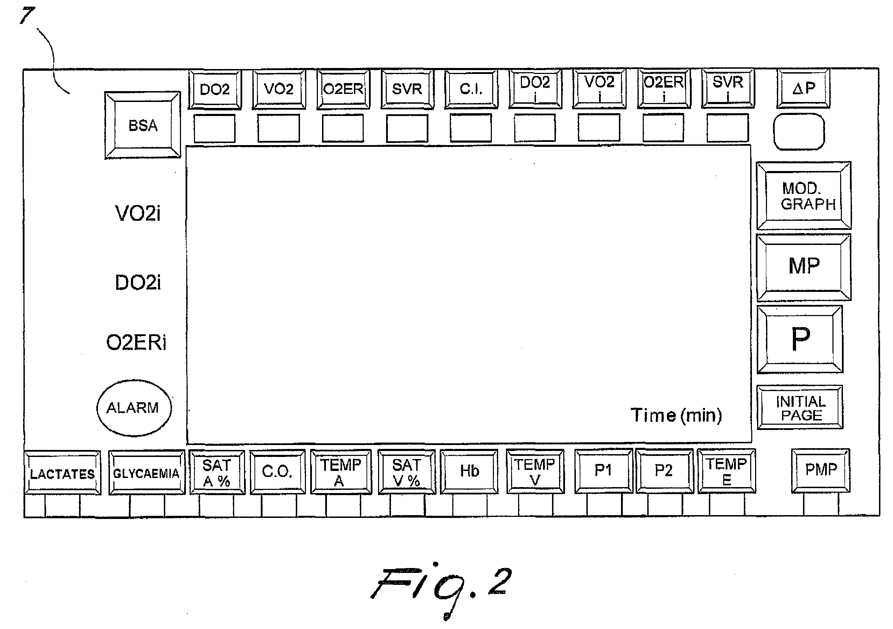

Let us now begin the description of the first embodiment of the invention considering the figure 1, which shows in schematic form an extracorporeal circulation circuit with the device according to the invention, and the figure 2, which shows one among the various images that can appear on the display screen of the monitoring appliance, or simply monitor, as it will be called henceforth, included in said device, according to what is shown in the description of operation.

The said image stands out because in it appear all the quantities which the device in the first embodiment being described takes into consideration, thereby being particularly significant: it is precisely for this reason that this image is identified by the symbol MS1, which stands for "main screen".

The same image also enables us to indicate how, on the display screen, two forms of representation appear of the data coming from the micro processor included in the device according to the invention: a representation in numerical form in the boxes at the top and bottom edges, and a representation in graphic form in the central field.

Returning to the figure 1, as will be noted, this represents the extracorporeal circulation circuit with a venous line 1 which conveys the blood from the patient 2 to the oxygenator 3, and an arterial line 4 which conveys the blood back to the patient.

The device according to the invention comprises the monitor globally indicated by 5, equipped with the micro processor 6 connected by means of the indicated dash and dot lines to the sensors which we shall immediately describe in detail, and which is also equipped with the display screen 7.

Now let us go on to describe the sensors present in the device, with the indication of the quantities which are continuously measured by each of them with consequent sending of the related data to the micro processor 6.

The first sensor is that indicated by 8 fitted to the patient 2 to measure his/her average PMP pressure which appears in the first box on the right at the bottom edge of the display screen.

This sensor 8 is of the type of a pressure transducer which is fitted to the patient in order to read his/her arterial pressure and consequently both the systolic pressure and the diastolic pressure. The sensor 8 is operatively connected to the micro processor 6, which is programmed to calculate the average PMP pressure of the patient on the basis of an algorithm of a type known to the technician of the sector. For example, the micro processor 6 is suitable for calculating the average PMP pressure on the basis of the maximum and minimum pressures read by the sensor 8 within a preset time interval.

The sensor 9 and the sensor 10 measure the lactates and the glycaemia respectively, and the related data appear in the first two boxes on the left on the lower edge of the display screen.

The three sensors 11, 12, 13 are suitable for measuring the three quantities "arterial saturation of the oxygen", blood flow and arterial blood temperature respectively, and the related data appear in the group of three boxes marked SAT A, C.O., TEMP A on the lower edge of the display screen.

The "optional flow sensor" 14 will be fitted in the circuit in a position distant from the sensor 12, and the data read by it prevail, if it is connected to the micro processor 6, over the data coming from the sensor 12, and will therefore appear in the related box of the display screen.

The three sensors 15, 16, 17 are suitable for measuring the three quantities "venous saturation of the oxygen", haemoglobin and venous blood temperature respectively, and the related data appear in the group of three boxes marked SAT V, Hb, TEMP V on the lower edge of the display screen.

The sensors 18 and 19 are installed at distant points of the circuit for measuring the blood pressure, and the related data appear in the two boxes at the lower edge of the display screen marked P1 and P2.

Finally, the sensor 20 is suitable for further measuring the blood temperature, and the related data appear in the box marked TEMP E at the lower edge of the display screen.

At this point, it is worth noting that the pair of sensors 11, 13 and the pair of sensors 15, 17 are necessarily meant to be connected on the arterial line and on the venous line of the circuit respectively, being able to be fitted at any points of the lines themselves or integrated in respective probes integrated in the structure of the oxygenator, while all the other sensors, with the sole obvious exception of the sensor which measures the average pressure of the patient, can be fitted indifferently at any points of the circuit.

Preferably, the sensor 15 is fitted in correspondence to the inlet connector of the venous line 1 in the oxygenator 3 together with one of the two sensors 12, 16, while the sensor 11 is fitted in correspondence to the outlet connector of the arterial line 4 from the oxygenator 3 together with the other of the two sensors 16, 12. Alternatively, the sensors 12 and 16 can be fitted indifferently at any points of the lines 1, 4.

According to the invention, the micro processor 6 is suitable for receiving the measured data at least from the sensors 11, 12, 15, 16 and the data entered by the operators by means of the relative entering means, making them appear at least in part on the display screen 7 and processing them, calculating at least some of the quantities deducible from them, making them appear on the display screen itself. The micro processor 6 is programmed to display in numerical form on the display screen 7 the values of all the quantities measured and calculated, and in graphic form, i.e., by means of diagrams, at least some of such values. Advantageously, the micro processor 6 has means suitable for calculating the quantities derivable from the detected quantities and from those entered by the operator.

More in detail, the micro processor 6 comprises means suitable for calculating at least one of the following quantities chosen from the group comprising: the quantity known by the code BSA relating to the corporeal surface of the patient, the quantity known by the code D02 relating to the quantity of transported oxygen, the quantity known by the code V02 relating to the quantity of oxygen consumed at tissue level, the quantity known by the code 02ER relating to the fraction of extraction of the oxygen, the quantity known by the code SVR relating to the systemic vascular resistance, the quantity known by the code C.I. relating to the cardiac index, the quantity known by the code ΔP relating to the difference between the two pressure values.

Preferably, the micro processor 6 also comprises means suitable for creating an archive of data relating to various monitoring procedures.

Furthermore, the micro processor 6 comprises means suitable for creating an archive of different perfusion programmes.

The conclusion of all the above is that the boxes at the lower edge of the display screen 7 relate to quantities measured continuously by the sensors described with sending of relevant data to the micro processor 6 and subsequent transfer to the display screen itself.

At the upper edge of the display screen are boxes each relating to quantities calculated by the micro processor 6, and the first box is that identified by the code BSA relating to the patient's corporeal surface, which is calculated on the basis of data which are entered by the operator and consist of the patient's weight and height.

All the other boxes relate to quantities calculated by the micro processor on the basis of data received from the sensors described above, as we shall now indicate in detail.

- D02, quantity of transported oxygen; calculated according to the formula

$$DO2 = C.O. \text{ x } (Hb \text{x} 1.34 \text{x} SAT\ A + K)$$

in which, we reiterate, the sensors provide the data relating to C.O., Hb, SAT A, and in which K represents a constant value representative of the difference between the pressure of the oxygen in the arterial and venous lines.

- V02, quantity of oxygen consumed at tissue level; it is calculated with the formula

$$VO2 = C.O. \text{ x } [(Hb \text{x} 1.34 \text{x} SAT \text{ A} + K) - (Hb \text{x} 1.34 \text{x} SAT \text{ V} + K)]$$

wherein the sensors provide the data relating to C.O., Hb, SAT A, SAT V, and wherein K represents a constant value according to what is specified above.
- 02ER, extraction fraction of the oxygen; it is calculated with the formula

$$O2ER = VO2/DO2$$

- The SVR quantity, systemic vascular resistance, is also calculated and this represents the load loss in the patient's vessels, variable according to his/her condition.
- C,I., cardiac index, is calculated on the basis of the BSA value, corporeal surface, deducted in turn, as has already been said above, from the knowledge of the "weight and height" data entered in the micro processor.
- On the basis of the C.I., the previously-quoted calculated quantities are recalculated in indexed form obtaining the quantities D02i, V02i, 02ERi, SVRi.

More in particular, the indexed quantities D02i, V02i, 02ERi and SVRi correspond to the quantities D02, V02, 02ER and SVR respectively, split up by the body surface of the BSA patient.

- $\Delta P$ is calculated as the difference between the P1 and P2 values provided by the sensors.

Finally, we observe the following.

The analytical exam performed so far has made possible a complete acquaintance with all the quantities taken into consideration by the device in the described embodiment, having seen these reflected in numerical representation form in the boxes at the upper and lower edges of the display screen in the image in figure 2, and it is therefore evident that the continuous monitoring of the quantities themselves, in large quantity, provides operators with a mass of information in real time (the updating of the values appearing on the display screen occurs very quickly, e.g., five seconds), which permits optimal patient management.

For this purpose, the graphic representation is also important of a number of quantities chosen from among those which appear represented in numerical form according to what has been previously illustrated, and by way of example only, in the display screen image in figure 2, the pattern of the three quantities DO2i, V02i and 02ERi appears in the form of a diagram according to time. But this, we repeat, is only an example, and in this respect, we refer to the description of the operation which immediately follows dedicated to illustrating the operating options provided to operators on the basis of the measurements taken by means of the described sensors.

Advantageously, the micro processor 6 also has means suitable for triggering an alarm signal relating to quantities selected by the operator when the values of same are outside an acceptable range, following the entering of the relevant maximum and minimum values by the operator.

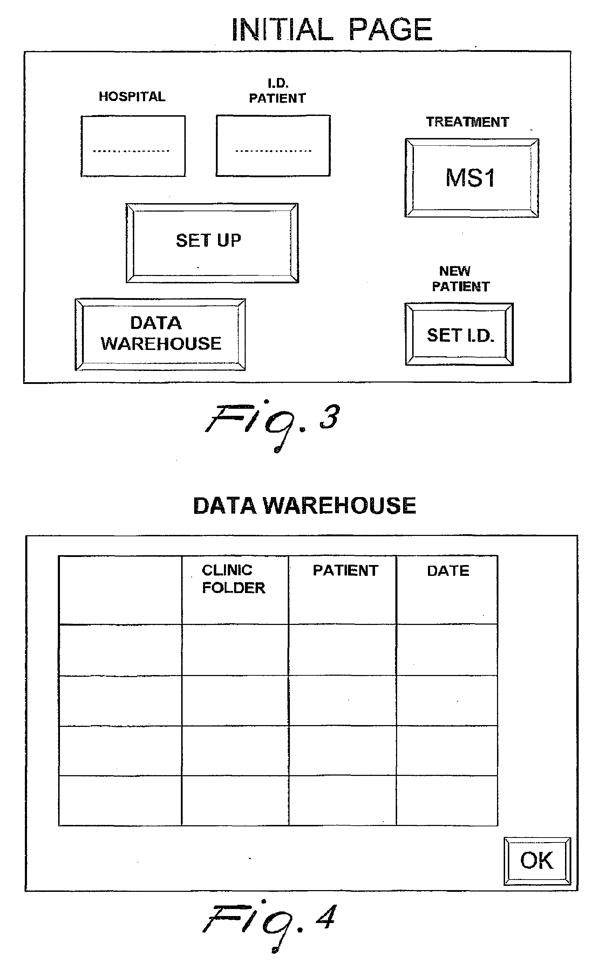

While the surgical operation is being prepared, the monitor 5 is switched on and, on the display screen 7, the INITIAL PAGE appears which is visible in figure 3.

If the DATA WAREHOUSE key is pressed, the image in figure 4 appears on the display screen with monitoring data previously obtained from other patients, which can be used for reference purposes; after selecting the data of interest, the OK key must be pressed and the "main screen" image of the selected case appears, which we previously called MS1.

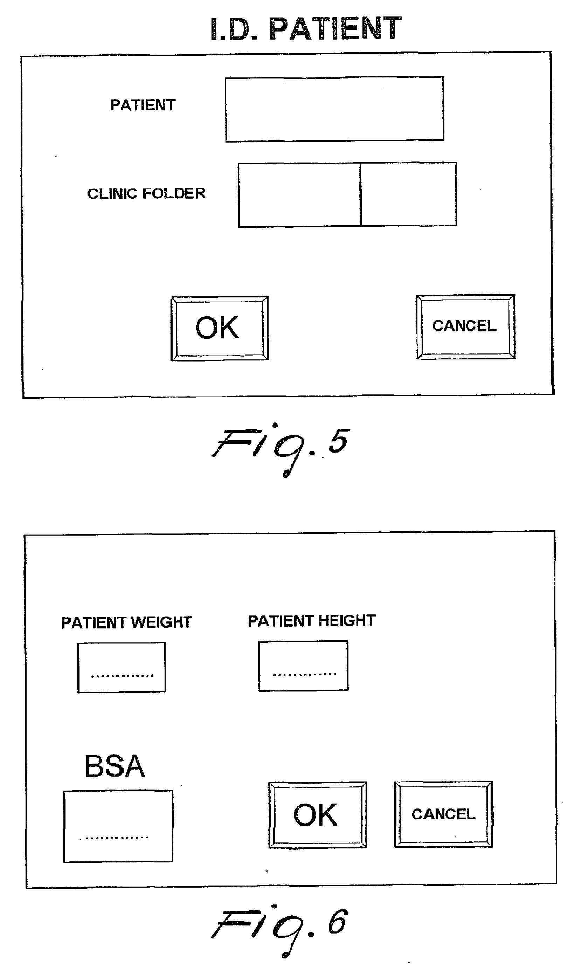

After having studied this MS1, the "INITIAL PAGE" key is pressed and the image of figure 3 reappears in which the SET I.D. key is pressed to make the ID PATIENT image of figure 5 appear.

After entering the data, the OK key must be pressed to return to the INITIAL PAGE of figure 3 in which the MS1 key is pressed.

The image of figure 2 thus appears: the display screen 7 is clean and the micro processor 6 is ready to receive data.

Before extracorporeal circulation starts, the operator in the MS1 of figure 2 presses the BSA key, and the image of figure 6 thus appears; after the patient's weight and height data have been entered using the keyboard, the calculated BSA value appears.

The operator presses the OK key, thus returning to the image of figure 2 in which the calculated BSA value will appear.

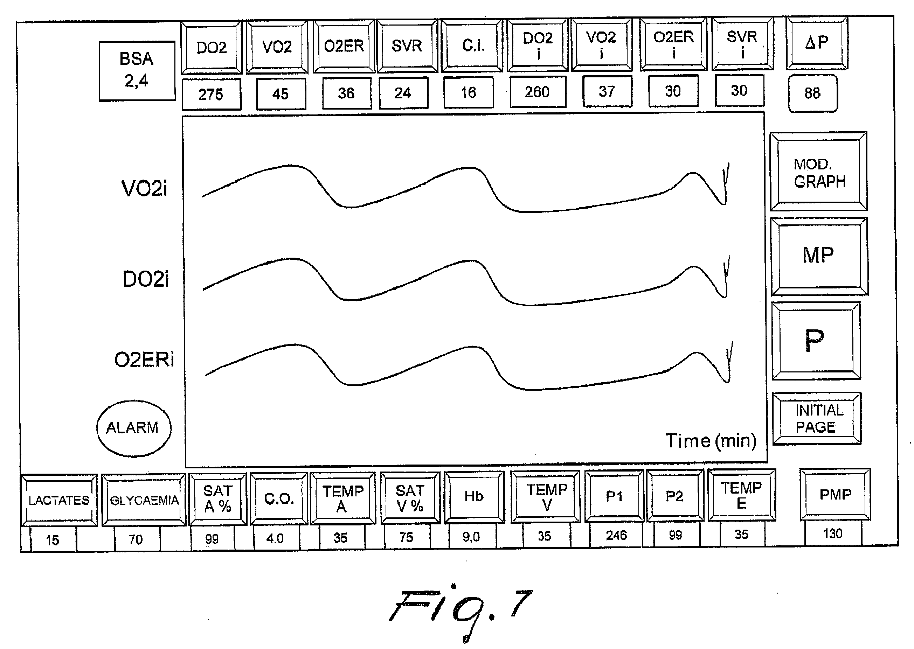

Now the extracorporeal circulation can begin, and with it the monitoring, thanks to the transmission of data to the micro processor by all the sensors described above: at a certain point of operation, the display screen will show an image of the MS1 like that of figure 7, with the graphs and the instantaneous values of the various quantities monitored in the single boxes, updated for example, as previously said, every five seconds.

Advantageously, the micro processor 6 has means for displaying, on the display screen 7, in graphic form, at least one quantity by means of diagram of same, which is represented on the axis of ordinates, as a function of time, which is represented on the axis of abscissas.

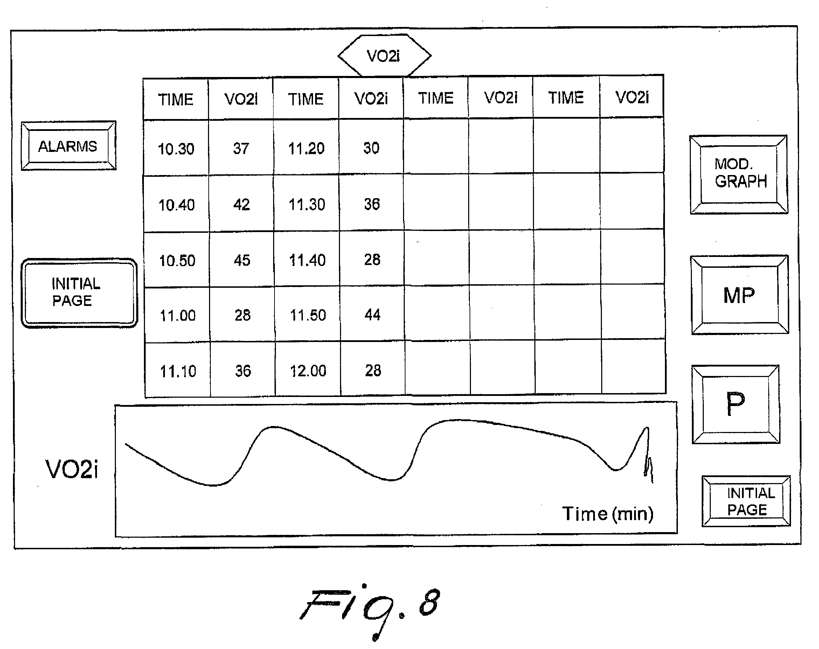

At a certain point, the operator could be interested in knowing, in particular, the V02i pattern according to time: he/she then presses the V02i key and the image of figure 8 appears on the display screen.

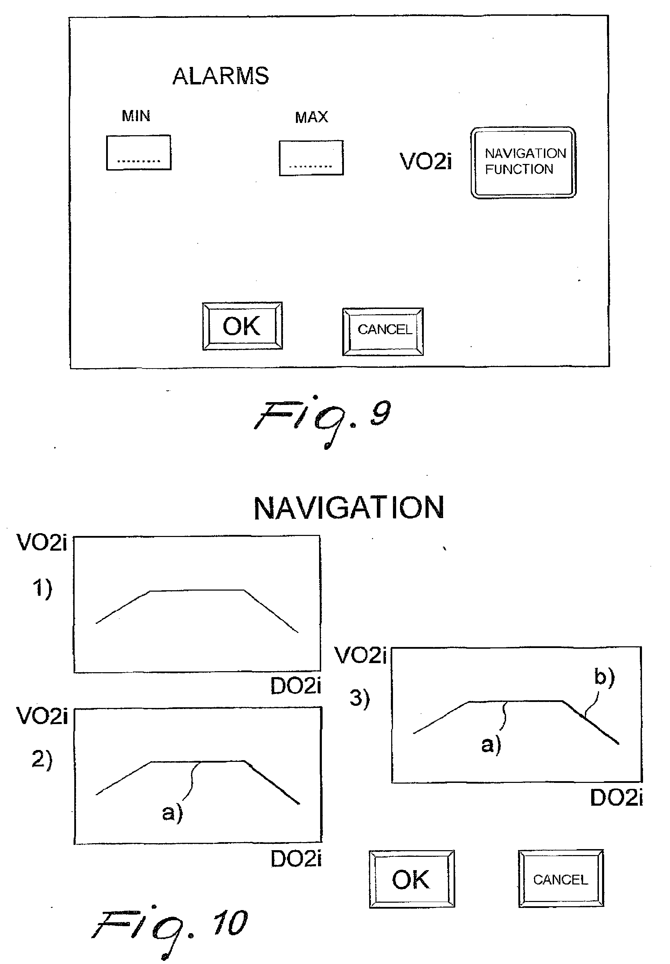

If it is then decided that the quantity V02i ought to be fitted with an alarm, all the operator has to do is press the ALARM key in the image of figure 8 so as to make the image of figure 9 appear in which the min and max value are entered by means of keyboard.

By pressing the OK key, return is made to the image of figure 8, and by pressing the V02i key, return is made to the MS1 of figure 7.

But a different type of alarm can be entered based on the following considerations.

More in particular, the micro processor 6 comprises means for displaying a diagram on the display screen 7 having in abscissa the quantity D02i and in ordinate the quantity V02i.

If the graph itself shows an increase of V02i with the increase of D02i this means that the consumption of oxygen by the patient is higher than that supplied to him/her, if the graph shows a constant pattern of V02i with the increase of D02i, a perfect balance exists between the oxygen consumed and that supplied, and finally, if the graph shows a drop in V02i with the increase of D02i, this means that more oxygen than necessary is being supplied in what is in any case a safety situation.

Now, to start this different form of alarm, suffice it to make a diagram appear on the display screen having, as said, the quantity D02i in abscissa and the quantity V02i in ordinate, and assess it on the basis of the above considerations, but with, over and above this, a possibility of colour highlighting according to what is now illustrated.

Preferably, the display means are programmed to represent the lines indicating an increase in V02i with an increase of D02i with a first colour, e.g., red, so as to highlight a dangerous situation and to represent the lines indicating a constant pattern of VO2i with the increase of D02i with a second colour different from the first colour, e.g., green, so as to highlight a safe situation.

Suitably, depending on preferences, the display means can be programmed to represent the lines of the diagram which indicate a drop in V02i with the increase of DO2i with the first or with the second colour.

This colour variation in accordance with the pattern of V02i with the variation of D02i permits providing the medical staff performing the operation with immediate and easy-to-understand information relating to the relation between the oxygen consumed by the patient and that supplied to the patient, without necessarily having to observe the graph pattern in detail. Such graphic representation is therefore intuitive and of immediate interpretation, technically contributing to facilitate the work of the medical operator.

The embodiment shown in figure 10 is described in more detail below.

In the image of figure 9, the NAVIGATION FUNCTION key is pressed, and the image of figure 10 appears showing three symbols, and more precisely the symbol 1) with a completely black line, the symbol 2) with the section of line a) green, the symbol 3) with the sections of lines a) and b) green.

If the symbol 1) is pressed, the graph mentioned with D02i and V02i in abscissa and ordinate respectively will appear completely with black line, the operator having waived colour highlighting, if the symbol 2) is pressed, said graph will appear with green horizontal lines, if, finally, the symbol 3) is pressed, the graph will appear with the lines horizontal and the lines descending with the increase of DO2i with the green colour.

If, in the image in figure 10, the OK key is pressed, the image of figure 9 reappears, if in this image the OK key is pressed, the image of figure 8 reappears, and if in this image the key V02i is pressed, the image of figure 7 reappears.

Alarms can be entered relating to other quantities only at min and max value level, operating as follows.

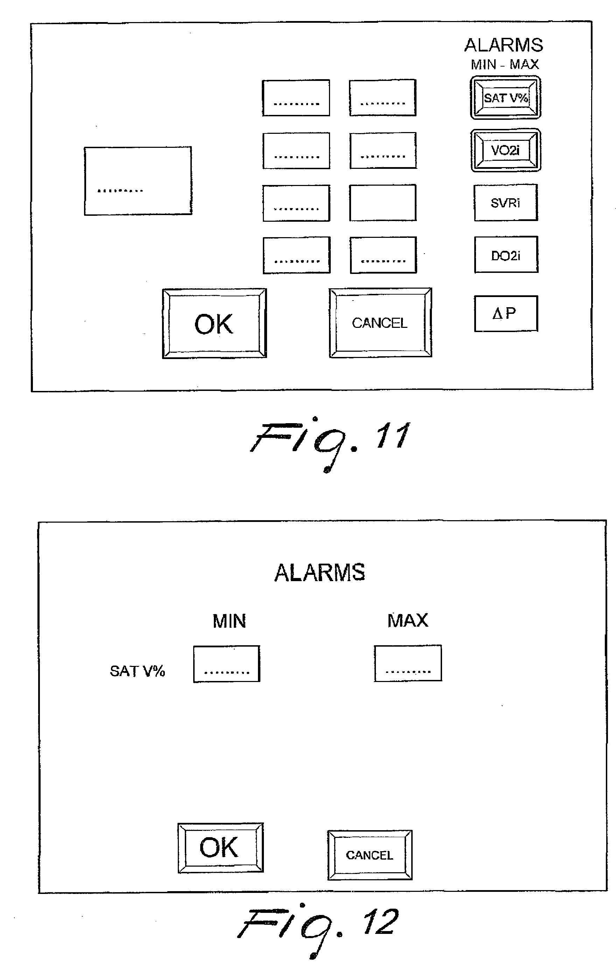

In the image of figure 7, the INITIAL PAGE key is pressed and the image of figure 3 appears, in which the SET UP key is pressed so as to make the image of figure 11 appear, which shows the list of alarms which can be entered.

One of the keys of the ALARMS column is pressed, e.g., the key marked SAT V, and the image of figure 12 appears.

After entering the min and max values with the keyboard, the path is completed in the opposite direction: when the OK key of the image of figure 12 is pressed, the image of figure 11 appears in which the OK key is pressed to make the image of figure 3 appear; when the MS1 key is pressed in this, the image of figure 7 reappears.

After concluding the description of the operating options relating to entering alarms, we must recall that the form of the image we have identified by MS1 shown in the figures 2 and 7, which shows three quantities displayed by graph according to the time, is just one of the options provided by the device, here supplied as an example, changeable by reducing or increasing the quantities displayed by graph according to the operator's requirements using the procedure now described.

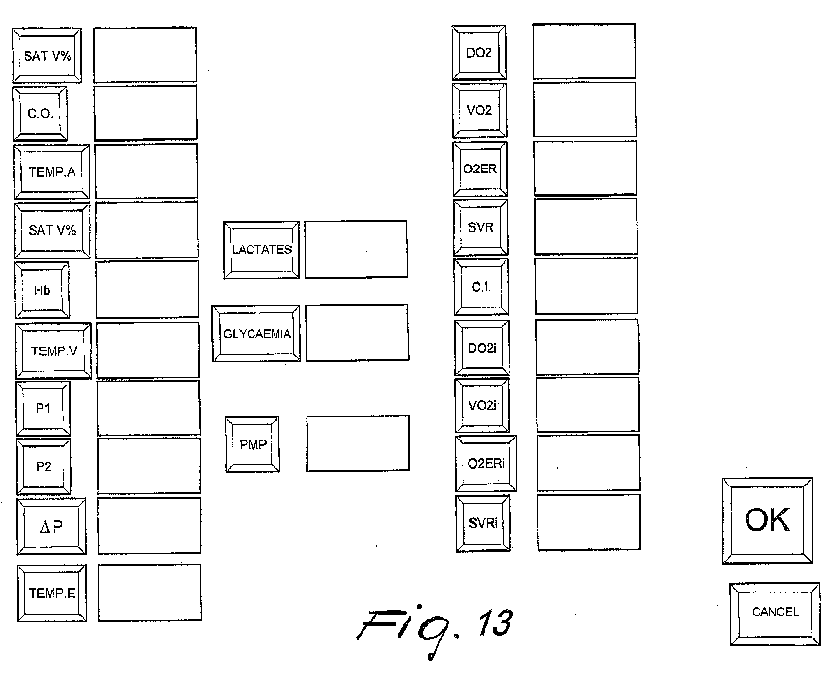

After pressing the MOD GRAPH key in figure 7, the image of figure 13 appears on the display screen, comprising all the MS1 quantities.

After choosing at most five quantities, the OK key must be pressed, thus determining on the display screen the appearance of a new MS1 form in which appear the quantities chosen in graphic form, therefore from one to five graphs, obviously in addition to all the values in the boxes updated every five seconds. Now we shall complete the description of the functionalities of the keys present in the MS1 image visible in the figures 2 and 7, and not yet considered.

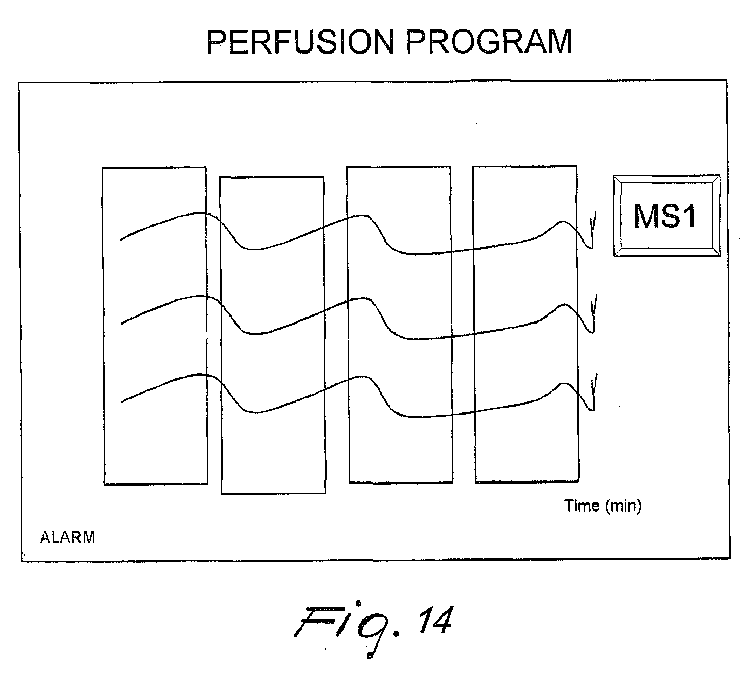

By pressing the P key, the image of the figure 14 appears, which illustrates a particular perfusion programme.

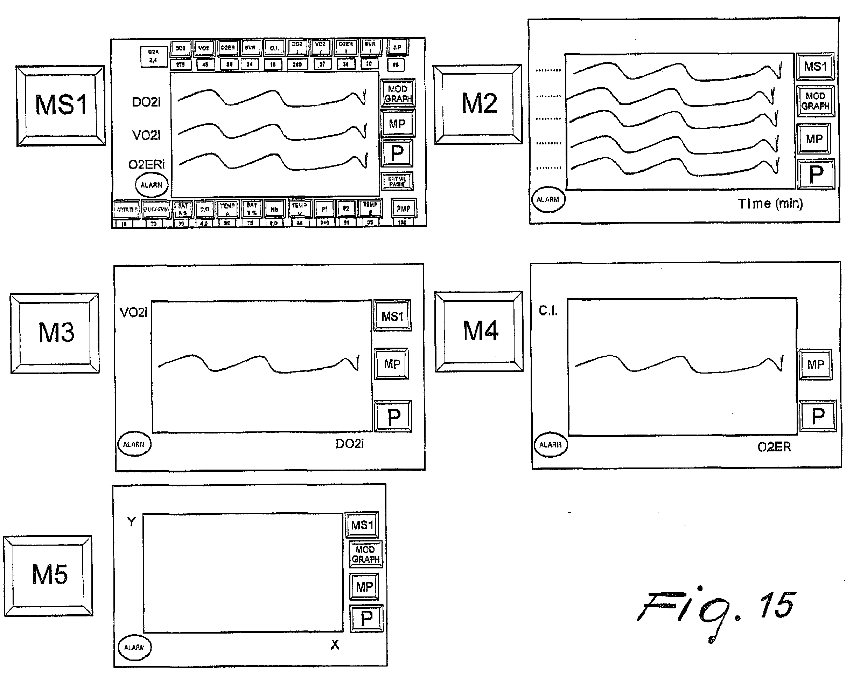

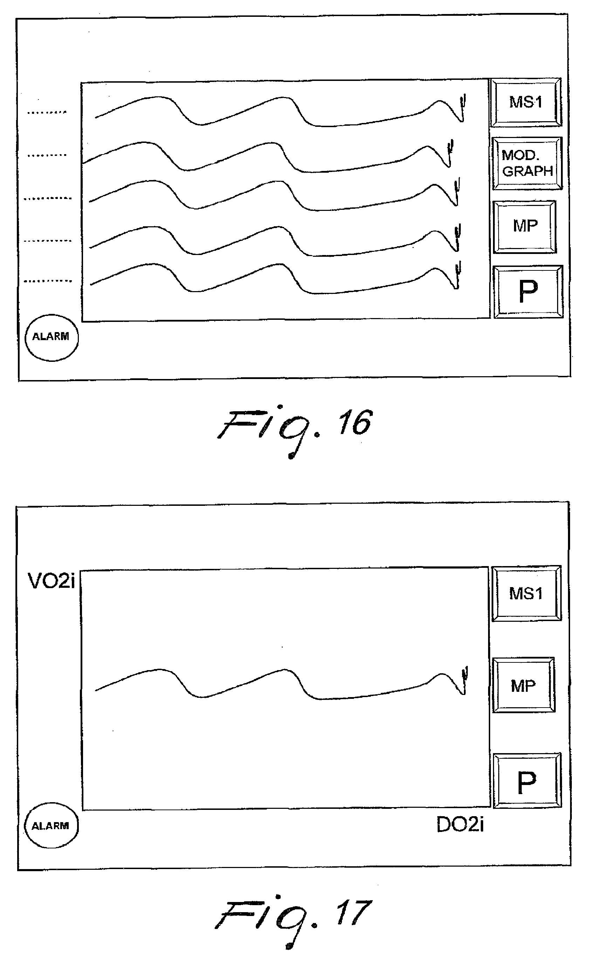

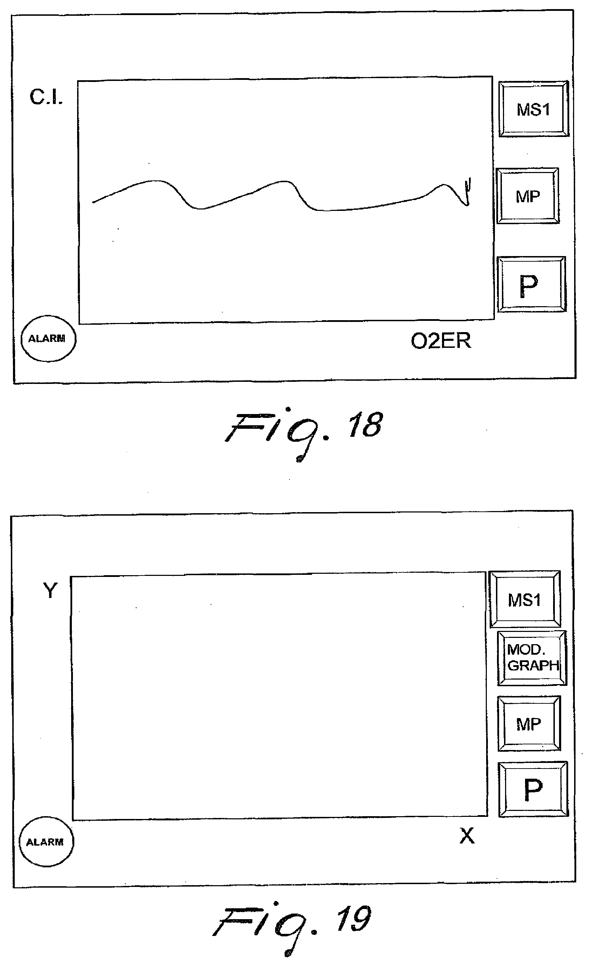

By pressing the MP key, the image of figure 15 appears which comprises miniaturised, besides the image of MS1, four other images of different graphs which can be referred to in enlarged form by pressing the relative keys M2, M3, M4, M5 obtaining the images of the figures 16, 17, 18, 19 respectively.

In this respect, we observe the following.

In the figure 16 M2 comprises graphs of quantities included among those listed in figure 13.

In figure 17, the graph M3 appears, mentioned with respect to the NAVIGATION FUNCTION alarm.

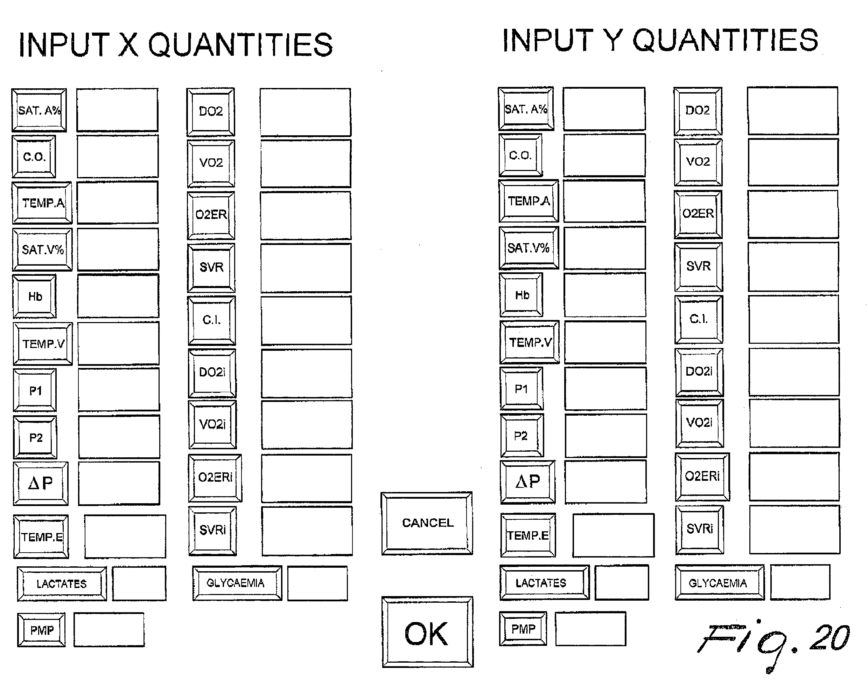

In figure 19, a graph M5 appears wherein the abscissa and the ordinate x and y will be selected from among the quantities of figure 20.

The description thus ends of the first embodiment of the device according to the invention which, as we have seen, has a large number of monitored quantities and various options for processing the quantities themselves, with entering of alarms and different displays on the display screen in addition to the main image, the "main screen" MS1, wherein appear all the quantities taken into consideration.

The possibility obviously exists of making devices dedicated to monitoring a greater or smaller number of quantities compared to those described above, providing different options for processing the quantities themselves.

It is possible, for example, to make a device having two probes situated at the inlet and outlet of the oxygenator, each with three sensors for the continuous measurement of the quantities SAT A, C.O., TEMP A, and SAT V, Hb, TEMP V respectively, which appear in numerical form on the display screen of figures 21 and 22, and again means suitable for data to be entered by an operator such as those relating to the patient's weight and height, and which also has a micro processor able to calculate only the quantities BSA, VO2, C.I., VO2i, represented on the display screen in numerical form, and with graphic representation relating to the quantity V02i only.

The described invention is susceptible to numerous other changes and variations, all falling within the scope of the inventive concept; furthermore, all the parts can be replaced with other technically equivalent elements.

**Claims**

1. Device for the continuous monitoring of characteristic blood quantities in extracorporeal circulation circuit, said circuit comprising a venous line (1) which conveys the blood from the patient (2) to an oxygenator device (3) and an arterial line (4) which conveys the blood from the oxygenator (3) back to the patient (2), comprising:

   - a sensor (11) connected to the venous line and a sensor (15) connected to the arterial line suitable for continuous measuring the quantities represented by venous saturation (SAT V) of the oxygen and by arterial saturation (SAT A) of the oxygen, respectively;
   - two sensors (12, 16) fitted in some points of the extracorporeal circuit, suitable for continuous measuring the quantities represented by blood flow (C.O.) and haemoglobin (Hb), respectively;
   - at least a sensor (18, 19) fitted in one point of the extracorporeal circuit, suitable for continuous measuring blood pressure;
   - means suitable for data entering by the operator;
   - a monitor (5) having a micro processor (6) and a display screen (7), said micro processor (6) being suitable for receiving the data measured by said sensors (11, 12, 15, 16, 18, 19) and data entered by the operator to make them appear at least in part on said display screen (7),

   **characterized by** the fact that it comprises at least two sensors (9, 10) fitted in one point of the extracorporeal circuit, suitable for continuous measuring lactates and glycaemia respectively, said micro processor (6) being suitable for lactates and glycaemia receiving the data measured by said sensors (9, 10) to make them appear on said display screen (7) in numerical form and possibly also in graphic form, by the fact that said microprocessor has means suitable for calculating the quantity of the corporeal surface of the patient (BSA), the quantity of transported oxygen (D02), the quantity of oxygen consumed at tissue level (V02), the quantity of the fraction of extraction of the oxygen (02ER), the quantity of the systemic vascular resistance (SVR), the quantity of the cardiac index (C.I.), the quantity of the difference between two pressure values (ΔP) measured by two of said blood pressure sensors (18, 19) installed at distant points of the circuit,, making them appear on the display screen (7), said micro processor (6) being programmed to display in numerical form on the display screen (7) the values of all the quantities measured and calculated, and in graphic form, at least some of such values,

that the micro processor (6) has means suitable for calculating at least one indexed quantity D02i, V02i, 02ERi, SVRi corresponding to the quantities D02, V02, 02ER and SVR respectively, split up by the corporeal surface of the patient (BSA),

and by the fact that the micro processor (6) comprises means for displaying a diagram having in abscissa the quantity D02i and in ordinate the quantity V02i, wherein said display means are programmed to represent the lines indicating an increase in V02i with an increase of D02i with a first colour so as to highlight a dangerous situation and to represent the lines indicating a constant pattern of V02i with the increase of D02i with a second colour, different from said first colour, so as to highlight a safe situation.

2. Device according to claim 1, **characterized by** the fact that the sensor (15) suitable for continuous measuring venous saturation (SAT V) of the oxygen is fitted at the inlet connector of the venous line (1) in the oxygenator (3) together with one of the two sensors (12, 16) suitable for continuous measuring blood flow (C.O.) and haemoglobin (Hb), while the sensor (11) suitable for continuous measuring arterial saturation (SAT A) of the oxygen is fitted at the outlet connector of the arterial line (4) from the oxygenator (3) together with the other of the two sensors (12, 16) suitable for continuous measuring blood flow (C.O.) and haemoglobin (Hb).

3. Device according to claim 1, **characterized by** the fact that the sensor (15) suitable for continuous measuring venous saturation (SAT V) of the oxygen is fitted at the inlet connector of the venous line (1) in the oxygenator (3), while the sensor (11) suitable for continuous measuring arterial saturation (SAT A) of the oxygen is fitted at the outlet connector of the arterial line (4) from the oxygenator (3), the sensors (12, 16) suitable for continuous measuring blood flow (C.O.) and haemoglobin (Hb) being fitted indifferently at any points of said lines (1, 4).

4. Device according to one or more of the preceding claims, **characterized by** the fact that it comprises a sensor (17) connected to the venous line (1) and a sensor (13) connected to the arterial line (4) suitable for continuous measuring quantities represented by the temperature of venous blood (TEMP V) and the temperature of arterial blood (TEMP A) respectively, said micro processor (6) being suitable for receiving the data measured by said sensors (13, 17) to make them appear on said display screen (7) in numerical form and possibly also in graphic form.

5. Device according to one or more of the preceding claims, **characterized by** the fact that it comprises a sensor (20) fitted in one point of the extracorporeal circuit, suitable for continuous measuring blood temperature (TEMP E), said micro processor (6) being suitable for receiving the data measured by said sensor (20) to make them appear on said display screen (7) in numerical form and possibly also in graphic form.

6. Device according to one or more of the preceding claims, **characterized by** the fact that it comprises a sensor (8) fittable to the body of the patient to read his/her arterial pressure and operatively connected to said micro processor (6), the latter being programmed to calculate the average pressure (PMP) of the patient according to the pressure values read by said sensor (8) within a preset time interval, to make the calculated data appear on said display screen (7) in numerical form and possibly also in graphic form.

7. Device according to one or more of the preceding claims, **characterized by** the fact that the micro processor (6) has means suitable for triggering an alarm signal relating to quantities selected by the operator when the values of same are outside an acceptable range, following the entering of the maximum and minimum values of said range by the operator.

8. Device according to one or more of the preceding claims, **characterized by** the fact that the micro processor (6) has means for displaying, on the display screen (7), in graphic form, at least one quantity by means of diagram of same, which is represented on the axis of ordinates, as a function of time, which is represented on the axis of abscissas.

9. Device according to one or more of the preceding claims, **characterized by** the fact that said display means are programmed to represent the lines of said diagram which indicate a drop in V02i with the increase of D02i with said second colour.

10. Device according to one or more of the preceding claims, **characterized by** the fact that the micro processor (6) has means for displaying a diagram having in abscissa the quantity 02ER, and in ordinate the quantity C.I.

**EP 2 455 116 B1**

**Patentansprüche**

1. Vorrichtung für die fortlaufende Überwachung charakteristischer Blutquantitäten in einem extrakorporalen Zirkulationskreislauf, wobei der Kreislauf eine venöse Leitung (1), die das Blut von dem Patienten (2) zu einer Oxygenatorvorrichtung (3) befördert, und eine arterielle Leitung (4), die das Blut von dem Oxygenator (3) zurück zu dem Patienten (2) befördert, umfasst, wobei die Vorrichtung Folgendes umfasst:

   - einen Sensor (11), der mit der venösen Leitung verbunden ist, und einen Sensor (15), der mit der arteriellen Leitung verbunden ist, geeignet zum fortlaufenden Messen der Quantitäten, die jeweils durch venöse Sättigung (SAT V) des Sauerstoffs und durch arterielle Sättigung (SAT A) des Sauerstoffs repräsentiert werden;
   - zwei Sensoren (12, 16), die in einigen Punkten des extrakorporalen Kreislaufs angebracht sind, geeignet zum fortlaufenden Messen der Quantitäten, die jeweils durch Blutfluss (C.O.) und Hämoglobin (Hb) repräsentiert werden;
   - wenigstens einen Sensor (18, 19), der in einem Punkt des extrakorporalen Kreislaufs angebracht ist, geeignet zum fortlaufenden Messen des Blutdrucks;
   - Mittel, geeignet zur Dateneingabe durch den Bediener;
   - einen Monitor (5), der einen Mikroprozessor (6) und einen Anzeigebildschirm (7) aufweist, wobei der Mikroprozessor (6) geeignet ist zum Empfangen der durch die Sensoren (11, 12, 15, 16, 18, 19) gemessenen Daten und der durch den Bediener eingegebenen Daten, um sie wenigstens zum Teil auf dem Anzeigebildschirm (7) erscheinen zu lassen,

   **dadurch gekennzeichnet, dass** sie wenigstens zwei Sensoren (9, 10) umfasst, die in einem Punkt des extrakorporalen Kreislaufs angebracht sind, geeignet zum fortlaufenden Messen von Laktaten beziehungsweise Glykämie, wobei der Mikroprozessor (6) geeignet ist zum Empfangen der Daten, die durch die Laktat- und Glykämiesensoren (9, 10) gemessen wurden, um sie in numerischer Form und möglicherweise ebenfalls in graphischer Form auf dem Anzeigebildschirm (7) erscheinen zu lassen, dadurch, dass der Mikroprozessor Mittel aufweist, die geeignet sind zum Berechnen der Quantität der Körperoberfläche des Patienten (BSA), der Quantität des transportierten Sauerstoffs (D02), der Quantität des auf Gewebeebene verbrauchten Sauerstoffs (V02), der Quantität des Anteiles der Extraktion des Sauerstoffs (02ER), der Quantität des systemischen Gefäßwiderstandes (SVR), der Quantität des Herzindex (C.I.), der Quantität der Differenz zwischen zwei Druckwerten (ΔP), gemessen durch zwei der an entfernten Punkten des Kreislaufs eingebauten Blutdrucksensoren (18, 19), um sie auf dem Anzeigebildschirm (7) erscheinen zu lassen, wobei der Mikroprozessor (6) dafür programmiert ist, in numerischer Form auf dem Anzeigebildschirm (7) die Werte aller gemessenen und berechneten Quantitäten und in graphischer Form wenigstens einige solcher Werte anzuzeigen, dass der Mikroprozessor (6) Mittel aufweist, geeignet zum Berechnen wenigstens einer indizierten Quantität D02i, V02i, 02ERi, SVRi, die den Quantitäten D02, V02, 02ER beziehungsweise SVR, geteilt durch die Körperoberfläche des Patienten (BSA), entsprechen, und dadurch, dass der Mikroprozessor (6) Mittel zum Anzeigen eines Diagramms umfasst, das in der Abszisse die Quantität D02i und in der Ordinate die Quantität V02i aufweist, wobei die Anzeigemittel dafür programmiert sind, die Linien, die eine Steigerung bei V02i mit einem Anstieg von D02i anzeigen, mit einer ersten Farbe darzustellen, um so eine gefährliche Situation hervorzuheben, und die Linien, die ein konstantes Muster von V02i mit dem Anstieg von D02i anzeigen, mit einer zweiten, von der ersten Farbe verschiedenen, Farbe darzustellen, um so eine sichere Situation hervorzuheben.

2. Vorrichtung nach Anspruch 1, **dadurch gekennzeichnet, dass** der Sensor (15), der zum fortlaufenden Messen der venösen Sättigung (SAT V) des Sauerstoffs geeignet ist, an dem Einlassverbinder der venösen Leitung (1) in dem Oxygenator (3) zusammen mit einem der zwei Sensoren (12, 16) angebracht ist, die zum fortlaufenden Messen von Blutfluss (C.O.) und Hämoglobin (Hb) geeignet sind, während der Sensor (11), der zum fortlaufenden Messen der arteriellen Sättigung (SAT A) des Sauerstoffs geeignet ist, an dem Auslassverbinder der arteriellen Leitung (4) von dem Oxygenator (3) zusammen mit dem anderen der zwei Sensoren (12, 16), die zum fortlaufenden Messen von Blutfluss (C.O.) und Hämoglobin (Hb) geeignet sind, angebracht ist.

3. Vorrichtung nach Anspruch 1, **dadurch gekennzeichnet, dass** der Sensor (15), der zum fortlaufenden Messen der venösen Sättigung (SAT V) des Sauerstoffs geeignet ist, an dem Einlassverbinder der venösen Leitung (1) in dem Oxygenator (3) angebracht ist, während der Sensor (11), der zum fortlaufenden Messen der arteriellen Sättigung (SAT A) des Sauerstoffs geeignet ist, an dem Auslassverbinder der arteriellen Leitung (4) von dem Oxygenator (3) angebracht ist, wobei die Sensoren (12, 16), die zum fortlaufenden Messen von Blutfluss (C.O.) und Hämoglobin (Hb) geeignet sind, indifferent an beliebigen Punkten der Leitungen (1, 4) angebracht sind.

4. Vorrichtung nach einem oder mehreren der vorhergehenden Ansprüche, **dadurch gekennzeichnet, dass** sie einen Sensor (17), der mit der venösen Leitung (1) verbunden ist, und einen Sensor (13), der mit der arteriellen Leitung (4) verbunden ist, geeignet zum fortlaufenden Messen der Quantitäten, die jeweils durch die Temperatur des venösen Bluts (TEMP V) und die Temperatur des arteriellen Bluts (TEMP A) repräsentiert werden, umfasst, wobei der Mikroprozessor (6) geeignet ist zum Empfangen der durch die Sensoren (13, 17) gemessenen Daten, um sie in numerischer Form und möglicherweise ebenfalls in graphischer Form auf dem Anzeigebildschirm (7) erscheinen zu lassen.

5. Vorrichtung nach einem oder mehreren der vorhergehenden Ansprüche, **dadurch gekennzeichnet, dass** sie einen Sensor (20) umfasst, der in einem Punkt des extrakorporalen Kreislaufs angebracht ist und der zum fortlaufenden Messen der Bluttemperatur (TEMP E) geeignet ist, wobei der Mikroprozessor (6) geeignet ist zum Empfangen der durch den Sensor (20) gemessenen Daten, um sie in numerischer Form und möglicherweise ebenfalls in graphischer Form auf dem Anzeigebildschirm (7) erscheinen zu lassen.

6. Vorrichtung nach einem oder mehreren der vorhergehenden Ansprüche, **dadurch gekennzeichnet, dass** sie einen Sensor (8) umfasst, der an den Körper des Patienten anpassbar ist, um seinen/ihren arteriellen Druck abzulesen, und der wirksam mit dem Mikroprozessor (6) verbunden ist, wobei der letztere dafür programmiert ist, den durchschnittlichen Druck (PMP) des Patienten entsprechend den Druckwerten berechnen, die durch den Sensor (8) innerhalb eines voreingestellten Zeitraums abgelesen wurden, um die berechneten Daten in numerischer Form und möglicherweise ebenfalls in graphischer Form auf dem Anzeigebildschirm (7) erscheinen zu lassen.

7. Vorrichtung nach einem oder mehreren der vorhergehenden Ansprüche, **dadurch gekennzeichnet, dass** der Mikroprozessor (6) Mittel aufweist, die geeignet sind zum Auslösen eines Warnsignals in Bezug auf durch den Bediener ausgewählte Quantitäten, wenn die Werte derselben außerhalb eines zulässigen Bereichs liegen, anschließend an das Eingeben der maximalen und minimalen Werte des Bereichs durch den Bediener.

8. Vorrichtung nach einem oder mehreren der vorhergehenden Ansprüche, **dadurch gekennzeichnet, dass** der Mikroprozessor (6) Mittel zum Anzeigen wenigstens einer Quantität in graphischer Form mit Hilfe eines Diagramms derselben auf dem Anzeigebildschirm (7) aufweist, die auf der Ordinatenachse dargestellt wird, als eine Funktion der Zeit, die auf der Abszissenachse dargestellt wird.

9. Vorrichtung nach einem oder mehreren der vorhergehenden Ansprüche, **dadurch gekennzeichnet, dass** die Anzeigemittel dafür programmiert sind, die Linien, die einen Abfall von V02i mit dem Anstieg von D02i anzeigen, mit der zweiten Farbe darzustellen.

10. Vorrichtung nach einem oder mehreren der vorhergehenden Ansprüche, **dadurch gekennzeichnet, dass** der Mikroprozessor (6) Mittel zum Anzeigen eines Diagramms aufweist, das in der Abszisse die Quantität 02ER und in der Ordinate die Quantität C.I. aufweist.

## Revendications

1. Dispositif pour la surveillance en continu de quantités sanguines caractéristiques dans un circuit de circulation extracorporelle, ledit circuit comprenant une ligne veineuse (1) qui achemine le sang depuis le patient (2) vers un dispositif oxygénateur (3) et une ligne artérielle (4) qui achemine le sang depuis l'oxygénateur (3) vers le patient (2), comprenant :

   - un capteur (11) branché sur la ligne veineuse et un capteur (15) branché sur la ligne artérielle aptes à mesurer en continu les quantités représentées par la saturation veineuse (SAT V) en oxygène et par la saturation artérielle (SAT A) en oxygène, respectivement ;
   - deux capteurs (12, 16) montés en certains points du circuit extracorporel, aptes à mesurer en continu les quantités représentées par le flux sanguin (C.O.) et l'hémoglobine (Hb), respectivement ;
   - au moins un capteur (18, 19) monté en un point du circuit extracorporel, apte à mesurer en continu la pression artérielle ;
   - des moyens appropriés pour la saisie de données par l'opérateur ;
   - un moniteur (5) équipé d'un microprocesseur (6) et d'un écran d'affichage (7), ledit microprocesseur (6) étant apte à recevoir les données mesurées par lesdits capteurs (11, 12, 15, 16, 18, 19) et les données saisies par l'opérateur afin de les afficher au moins partiellement sur ledit écran d'affichage (7),

**caractérisé en ce qu'**il comprend au moins deux capteurs (9, 10) montés en un point du circuit extracorporel, aptes à mesurer en continu les lactates et la glycémie respectivement, ledit microprocesseur (6) étant apte à recevoir les données mesurées par lesdits capteurs pour les lactates et la glycémie (9, 10) afin de les afficher sur ledit écran d'affichage (7) sous forme numérique et éventuellement aussi sous forme graphique, **en ce que** ledit microprocesseur possède des moyens aptes à calculer la quantité de la surface corporelle du patient (BSA), la quantité d'oxygène transporté (D02), la quantité d'oxygène consommé au niveau tissulaire (V02), la quantité de la fraction d'extraction de l'oxygène (02ER), la quantité de la résistance vasculaire systémique (SVR), la quantité de l'index cardiaque (C.I.), la quantité de la différence entre deux valeurs de pression (ΔP) mesurées par deux desdits capteurs de pression artérielle (18, 19) installés à des points distants du circuit, les affichant sur l'écran d'affichage (7), ledit microprocesseur (6) étant programmé pour afficher sous forme numérique sur l'écran d'affichage (7) les valeurs de toutes les quantités mesurées et calculées, et sous forme graphique, au moins certaines de ces valeurs, que le microprocesseur (6) possède des moyens aptes à calculer au moins une quantité indexée D02i, V02i, 02ERi, SVRi correspondant aux quantités D02, V02, 02ER et SVR respectivement, rapportées à la surface corporelle du patient (BSA), et **en ce que** le microprocesseur (6) comprend des moyens pour afficher un diagramme ayant en abscisse la quantité D02i et en ordonné la quantité V02i, dans lequel lesdits moyens d'affichage sont programmés pour représenter les courbes indiquant une augmentation de V02i avec une augmentation de D02i par une première couleur afin de faire ressortir une situation dangereuse et pour représenter les courbes indiquant un motif constant de V02i avec l'augmentation de D02i par une seconde couleur, différente de la première couleur, afin de faire ressortir une situation de sécurité.

2. Dispositif selon la revendication 1, **caractérisé en ce que** le capteur (15) apte à mesurer en continu la saturation veineuse (SAT V) en oxygène est monté au niveau du raccord d'entrée de la ligne veineuse (1) dans l'oxygénateur (3) en association avec l'un des deux capteurs (12, 16) aptes à mesurer en continu le flux sanguin (C.O.) et l'hémoglobine (Hb), alors que le capteur (11) apte à mesurer en continu la saturation artérielle (SAT A) en oxygène est monté au niveau du raccord de sortie de la ligne artérielle (4) de l'oxygénateur (3) en association avec l'autre des deux capteurs (12, 16) aptes à mesurer en continu le flux sanguin (C.O.) et l'hémoglobine (Hb).

3. Dispositif selon la revendication 1, **caractérisé en ce que** le capteur (15) apte à mesurer en continu la saturation veineuse (SAT V) en oxygène est monté au niveau du raccord d'entrée de la ligne veineuse (1) dans l'oxygénateur (3), alors que le capteur (11) apte à mesurer en continu la saturation artérielle (SAT A) en oxygène est monté au niveau du raccord de sortie de la ligne artérielle (4) de l'oxygénateur (3), les capteurs (12, 16) aptes à mesurer en continu le flux sanguin (C.O.) et l'hémoglobine (Hb) étant montés indifféremment à n'importe quel point desdites lignes (1, 4).

4. Dispositif selon l'une ou plusieurs des revendications précédentes, **caractérisé en ce qu'**il comprend un capteur (17) branché sur la ligne veineuse (1) et un capteur (13) branché sur la ligne artérielle (4) aptes à mesurer en continu des quantités représentées par la température du sang veineux (TEMP V) et la température du sang artériel (TEMP A) respectivement, ledit microprocesseur (6) étant apte à recevoir les données mesurées par lesdits capteurs (13, 17) afin de les afficher sur ledit écran d'affichage (7) sous forme numérique et éventuellement aussi sous forme graphique.

5. Dispositif selon l'une ou plusieurs des revendications précédentes, **caractérisé en ce qu'**il comprend un capteur (20) monté à un point du circuit extracorporel, apte à mesurer en continu la température du sang (TEMP E), ledit microprocesseur (6) étant apte à recevoir les données mesurées par ledit capteur (20) afin de les afficher sur ledit écran d'affichage (7) sous forme numérique et éventuellement aussi sous forme graphique.

6. Dispositif selon l'une ou plusieurs des revendications précédentes, **caractérisé en ce qu'**il comprend un capteur (8) pouvant être adapté au corps du patient pour lire sa pression artérielle et connecté de façon opérationnelle audit microprocesseur (6), ce dernier étant programmé pour calculer la pression moyenne (PMP) du patient selon les valeurs de pression lues par ledit capteur (8) dans un intervalle de temps prédéfini, pour afficher les données calculées sur ledit écran d'affichage (7) sous forme numérique et éventuellement aussi sous forme graphique.

7. Dispositif selon l'une ou plusieurs des revendications précédentes, **caractérisé en ce que** le microprocesseur (6) possède des moyens aptes à déclencher un signal d'alarme par rapport à des quantités sélectionnées par l'opérateur lorsque les valeurs de celles-ci sont en dehors d'une plage acceptable, suite à la saisie des valeurs maximales et minimales de ladite plage par l'opérateur.

**8.** Dispositif selon l'une ou plusieurs des revendications précédentes, **caractérisé en ce que** le microprocesseur (6) possède des moyens pour afficher, sur l'écran d'affichage (7), sous forme graphique, au moins une quantité au moyen d'un diagramme de celle-ci, qui est représentée sur l'axe des ordonnées, en fonction du temps, qui est représenté sur l'axe des abscisses.

**9.** Dispositif selon l'une ou plusieurs des revendications précédentes, **caractérisé en ce que** lesdits moyens d'affichage sont programmés pour représenter les courbes dudit diagramme qui indiquent une chute en V02i avec l'augmentation de D02i par ladite seconde couleur.

**10.** Dispositif selon l'une ou plusieurs des revendications précédentes, **caractérisé en ce que** le microprocesseur (6) possède des moyens pour afficher un diagramme ayant en abscisse la quantité 02ER, et en ordonnée la quantité C.I.

Fig. 1

Fig.2

# INITIAL PAGE

HOSPITAL

I.D.
PATIENT

................

................

TREATMENT

MS1

SET UP

NEW
PATIENT

DATA
WAREHOUSE

SET I.D.

*Fig. 3*

# DATA WAREHOUSE

| | CLINIC FOLDER | PATIENT | DATE |
|---|---|---|---|
| | | | |
| | | | |
| | | | |
| | | | |

OK

*Fig. 4*

# I.D. PATIENT

PATIENT

CLINIC FOLDER

OK       CANCEL

## Fig.5

PATIENT WEIGHT      PATIENT HEIGHT

.............          .............

## BSA

.............      OK      CANCEL

## Fig.6

| BSA 2,4 | DO2 | VO2 | O2ER | SVR | C.I. | DO2 i | VO2 i | O2ER i | SVR i | ΔP |
|---------|-----|-----|------|-----|------|-------|-------|--------|-------|-----|
|         | 275 | 45  | 36   | 24  | 16   | 260   | 37    | 30     | 30    | 88  |

VO2i

DO2i

O2ERi

ALARM

MOD. GRAPH

MP

P

INITIAL PAGE

Time (min)

| LACTATES | GLYCAEMIA | SAT A % | C.O. | TEMP A | SAT V % | Hb | TEMP V | P1 | P2 | TEMP E | PMP |
|----------|-----------|---------|------|--------|---------|-----|--------|-----|-----|--------|-----|
| 15       | 70        | 99      | 4.0  | 35     | 75      | 9,0 | 35     | 246 | 99  | 35     | 130 |

*Fig. 7*

EP 2 455 116 B1

Fig. 8

ALARMS

MIN

MAX

........

.........

VO2i

NAVIGATION
FUNCTION

OK

CANCEL

*Fig. 9*

NAVIGATION

VO2i

1)

DO2i

VO2i

2)

a)

DO2i

VO2i

3)

b)

a)

DO2i

OK

CANCEL

*Fig. 10*

*Fig. 11*

*Fig. 12*

Fig. 13

PERFUSION PROGRAM

MS1

Time (min)

ALARM

Fig. 14

Fig. 15

*Fig. 16*

*Fig. 17*

C.I.

MS1

MP

P

ALARM

O2ER

*Fig. 18*

Y

MS1

MOD.
GRAPH

MP

P

ALARM

X

*Fig. 19*

# INPUT X QUANTITIES

| | | | |
|---|---|---|---|
| SAT. A% | | DO2 | |
| C.O. | | VO2 | |
| TEMP.A | | O2ER | |
| SAT.V% | | SVR | |
| Hb | | C.I. | |
| TEMP.V | | DO2i | |
| P1 | | VO2i | |
| P2 | | O2ERi | |
| ΔP | | SVRi | |
| TEMP.E | | | |
| LACTATES | | GLYCAEMIA | |
| PMP | | | |

# INPUT Y QUANTITIES

| | | | |
|---|---|---|---|
| SAT. A% | | DO2 | |
| C.O. | | VO2 | |
| TEMP.A | | O2ER | |
| SAT.V% | | SVR | |
| Hb | | C.I. | |
| TEMP.V | | DO2i | |
| P1 | | VO2i | |
| P2 | | O2ERi | |
| ΔP | | SVRi | |
| TEMP.E | | | |
| LACTATES | | GLYCAEMIA | |
| PMP | | | |

CANCEL

OK

*Fig. 20*

*Fig. 21*

*Fig. 22*

**EP 2 455 116 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4401431 A